# EUROPEAN PATENT APPLICATION

(11) **EP 1 327 443 A1**
(43) Date of publication of application: **16.07.2003**
(21) Application number: 02258923.8
(22) Date of filing: 20.12.2002
(51) Int. Cl.: A61K 31/198, A61K 33/06, A61K 33/42, A23L 1/305, A23L 1/304

(54) **Therapeutic or preventing agent for the diseases caused by a decrease in the expression level of the klotho protein**

(30) Priority: 21.12.2001 JP 2001389912
(71) Applicant: KYOWA HAKKO KOGYO CO., LTD., Chiyoda-ku, Tokyo 100-8185 (JP)
(72) Inventor: Kamiya, Toshikazu, c/o Tsukuba Research Lab., Tsukuba-shi, Ibaraki 305-0841 (JP); Morishita, Koji, c/o Tsukuba Research Laboratories, Tsukuba-shi, Ibaraki 305-0841 (JP); Katakura, Yuji, c/o Tsukuba Research Laboratories, Tsukuba-shi, Ibaraki 305-0841 (JP); Nabeshima, Yo-ichi, 173-901 Nishimaruto-cho, Kyoto-shi, Kyoto 602-8148 (JP)
(74) Representative: Harding, Charles Thomas

(57) **Abstract**

The present invention provides a therapeutic or preventing agent for diseases caused by a decrease in the expression level of Klotho protein in animals or humans and a feed or food and drink for the treatment or prevention of such diseases, which comprise ornithine or a salt thereof as an active ingredient. Also provided are a therapeutic or preventing agent for premature aging-like syndrome of homozygous klotho mutant animals and a feed for the treatment or prevention of the syndrome, which comprise ornithine or a salt thereof as an active ingredient, as well as a method for the treatment or prevention of premature aging-like syndrome of homozygous klotho mutant animals which comprises feeding the animals with the above feed.

## Description

### Background of the Invention

The present invention relates to a therapeutic or preventing agent for diseases caused by a decrease in the expression of Klotho protein, a feed or food and drink for the treatment or prevention of diseases caused by a decrease in the expression of Klotho protein, a therapeutic or preventing agent for premature aging-like syndrome of homozygous klotho mutant animals, a feed for the treatment or prevention of premature aging-like syndrome of homozygous klotho mutant animals, and a method for the treatment or prevention of premature aging-like syndrome of homozygous klotho mutant animals.

Klotho mutant mice are a mouse line exhibiting features resembling human premature aging which has been established by Kuroo, et al. [Nature, 390, 45 (1997)] by means of an insertion mutation. This mutation is recessive heredity, and homozygous individuals show growth similar to that of wild-type and heterozygous individuals during the suckling period. However, after the weaning period (3 weeks old), they begin to manifest various symptoms resembling aging, e.g., retardation in body weight increase, reduction in life span (less than about 9 weeks), ectopic calcification at arterial media and other tissues, abnormality in osteogenesis such as a decrease in bone density, thymic involution, missing of Purkinje cells, infertility by gonadal dysgenesis, skin atrophy accompanied by a decrease of subcutaneous fat and hair roots, abnormality in motor functions such as walking, and abnormality in pituitary function. All of these symptoms appear almost simultaneously by a defect in the expression of a klotho gene, which enables observation of various symptoms of aging in a short period of time with a single model system.

On the basis of the fact that homozygous individuals of klotho mutant mice show symptoms resembling human premature aging as described above, the klotho gene is acknowledged as an aging-suppressing gene. Therefore, it is considered that various klotho gene-associated diseases can be treated or prevented by increasing or decreasing the expression of klotho gene or Klotho protein.

Known methods for increasing or decreasing the expression of Klotho protein in animals include methods for decreasing the expression of Klotho protein by knockout of the gene encoding the protein itself or a gene of its expression regulatory region, and methods for increasing the expression of Klotho protein by transplantation of cells expressing Klotho protein or by gene manipulation.

As homozygous individuals of klotho mutant mice show symptoms resembling human premature aging, they will serve as important subjects in experiments for the elucidation of the mechanism of aging and experiments on the methods for suppressing or alleviating aging.

However, homozygous individuals of klotho mutant mice are not always adequate for the experiments for the elucidation of the mechanism of aging or the experiments on the methods for suppressing aging, because of their short life span. Particularly, there has not been provided an adequate aging model system of aged (mature) individuals, in spite of the high importance of such a model system.

Further, homozygous individuals of klotho mutant mice lack reproductive ability, and therefore, mating of pairs of heterozygous klotho mutant mice is necessary for the breeding and the maintenance of the line. Mice which are born as a result of such mating have the genotypes of wild-type, heterozygous and homozygous in 1:2:1 ratio in accordance with Mendel's law. That is, the number of homozygous individuals obtained is 1/4 of the total number of animals born, which is not sufficient for the purpose Thus, the difficulty in breeding is the chief obstacle to sufficient and stable supply of homozygous mice for the development of drugs and the research of aging. Moreover, in order to identify homozygous individuals, it is necessary to judge the genotype by polynucleotide chain reaction (PCR), which is a complicated method involving isolation of a gene from each individual, because the genotypes are difficult to distinguish with the naked eye up to about 3 weeks after birth.

WO 01/05244 discloses the following: a method for treating or preventing aging-associated diseases of an animal which comprises feeding the animal with a feed comprising a compound containing a divalent cationic metal and/or a compound containing phosphorus; a feed or food for the treatment or prevention of aging-aseociated diseases of an animal or human comprising a compound containing a divalent cationic metal and/or a compound containing phosphorus; a method for regulating the expression of Klotho protein in an animal which comprises feeding the animal with a feed comprising a compound containing a divalent cationic metal and/or a compound containing phosphorus; an animal in which the expression of Klotho protein is regulated and which is obtained by feeding of a feed comprising a compound containing a divalent cationic metal and/or a compound containing phosphorus; a feed for a homozygous klotho mutant animal which is effective for the inhibition or alleviation of premature aging-like syndrome of the homozygous klotho mutant animal when fed to the homozygous klotho mutant animal; a method for raising a homozygous klotho mutant animal which can prolong the life span of the homozygous klotho mutant animal by using said feed; a method for raising a homozygous klotho mutant animal which can confer reproductive ability on the homozygous klotho mutant animal; a method for breeding homozygous klotho mutant animals; a method for raising a homozygous klotho mutant animal which can inhibit or alleviate premature aging-like syndrome characteristic of homozygous klotho mutant animals; a method for regulating the expression level of Klotho protein in a homozygous klotho mutant animal; a method for treating or preventing a disease of a homozygous klotho mutant animal; and homozygous klotho mutant animals which are obtained by said methods.

An object of the present invention is to provide a therapeutic or preventing agent for diseases caused by a decrease in the expression of Klotho protein in animals or humans, a feed or food and drink for the treatment or prevention of diseases caused by a decrease in the expression of Klotho protein in animals or humans, a therapeutic or preventing agent for premature aging-like syndrome of homozygous klotho mutant animals, a feed for the treatment or prevention of premature aging-like syndrome of homozygous klotho mutant animals, and a method for the treatment or prevention of premature aging-like syndrome of homozygous klotho mutant animals.

### Summary of the Invention

The present invention relates to the following (1) to (12).
(1) A therapeutic or preventing agent for a disease caused by a decrease in the expression of Klotho protein in an animal or a human which comprises ornithine or a salt thereof as an active ingredient.
(2) The therapeutic or preventing agent according to (1), wherein the disease is an aging-associated disease.
(3) A feed or food and drink for the treatment or prevention of a disease caused by a decrease in the expression of Klotho protein in an animal or a human which comprises ornithine or a salt thereof as an active ingredient.
(4) The feed or food and drink according to (3), further comprising at least one compound selected from the group consisting of compounds containing a divalent cationic metal and compounds containing phosphorus.
(5) The feed or food and drink according to (3) or (4), wherein the disease is an aging-associated, disease.
(6) A therapeutic or preventing agent for premature aging-like syndrome of a homozygous klotho mutant animal which comprises ornithine or a salt thereof as an active ingredient.
(7) A feed for the treatment or prevention of premature aging-like syndrome of a homozygous klotho mutant animal which comprises ornithine or a salt thereof as an active ingredient.
(8) The feed according to (7), further comprising at least one compound selected from the group consisting of compounds containing a divalent cationic metal and compounds containing phosphorus,
(9) A method for treating or preventing premature aging-like syndrome of a homozygous klotho mutant animal which comprises feeding the animal with the feed according to (7) or (8).
(10) The method according to (9), wherein the animal is a homozygous klotho mutant mouse.
(11) Use of ornithine or a salt thereof for the preparation of a therapeutic or preventing agent for a disease caused by a decrease in the expression level of Klotho protein in an animal or a human.
(12) Use of ornithine or a salt thereof for the preparation of a feed or food and drink for a disease caused by a decrease in the expression level of Klotho protein in an animal or a human.

### Detailed Description of the Invention

In the present invention, there is no specific restriction as to the disease caused by a decrease in the expression of Klotho protein in animals or humans. Examples of such diseases include hereditary premature aging, aging, hypodynamia, parkinsonism-like gait disturbance, involution of external genitalia, gonadal involution, thyroid involution, ectopic calcification, abnormality in chondrogenesis, alopecia, skin involution, decrease of growth hormone-producing cells, involution of corpus luteum hormone- and follicle-stimulating hormone-producing cells, degeneration and decrease of Purkinje cells, arteriosclerosis, pulmonary emphysema, osteoporosis, hump back, abnormal formation of adipose tissue, abnormality in lipid metabolism, osteoarthritis, diabetes, diabetic complication, senile dementia of Alzheimer type, lowering of immune function, autoimmune diseases, cataract, hypertension, cerebral apoplexy, myocardial infarction, hyperlipemia, atrophic gastritis, decrease of gastric secretion, constipation, cholelithiasis, disorder of lipid digestion and absorption, dysgeusia, dysosmia, hypexphosphatemia, and hypoparathyroidism. Preferred are aging-associated diseases of animals or humans.

Examples of the aging-associated diseases of animals or humans include hypodynamia, involution of external genitalia, gonadal involution, thyroid involution, ectopic calcification, alopecia, skin involution, decrease of growth hormone-producing cells, involution of corpus luteum hormone- and follicle-stimulating hormone-producing cells, degeneration and decrease of Purkinje cells, arteriosclerosis, pulmonary emphysema, osteoporosis, hump back, involution of subcutaneous fat, abnormality in lipid metabolism, osteoarthritis, diabetes, diabetic complication, senile dementia of Alzheimer type, lowering of immune function, autoimmune diseases, cataract, hypertension, cerebral apoplexy, myocardial infarction, hyperlipemia, atrophic gastritis, decrease of gastric secretion, disorder of lipid digestion and absorption, dysgeusia, and dysosmia.

The animals that can be the subjects of the present invention are not specifically limited except for human, and include mammals, birds, reptiles, amphibians and fish. Specifically, the animals include livestock such as cows and pigs, poultry such as hens and turkeys, and pets such as dogs, cats and mice. The heterozygous klotho mutant animals described below are also included in the animals of the present invention.

The homozygous klotho mutant animals that can be the subjects of the present invention are homozygotes carrying a mutation in the gene encoding Klotho protein having aging-suppressing activity (klotho gene). The heterozygous klotho mutant animals are heterozygotes carrying a mutation in the klotho gene.

The Klotho protein and the gene encoding this protein mentioned above are described as the aging-suppressing polypeptide and the aging-suppressing gene, for example, in WO 98/29544.

The homozygous klotho mutant animals of the present invention can be obtained, for example, by the following method in the case of animals having the klotho gene of which the expression regulatory region is known. That is, after eggs or fertilized eggs are subjected to an arbitrary procedure for artificially inserting a foreign gene or introducing a partial deletion or a mutation into the klotho gene expression regulatory region, a part of organ (e.g. kidney) is taken from the generated individuals. The expression level of the klotho gene in the organ is determined by Northern blotting using the known klotho gene as a probe or Western blotting using an anti-Klotho protein antibody, and individuals with reduced expression level are selected.

Another example of the method for obtaining the homozygous klotho mutant animals is as follows. Eggs or fertilized eggs of various animals are subjected to an arbitrary procedure for artificially inserting a foreign gene or introducing a mutation such as a partial deletion, followed by mating of the generated transgenic animals. After a part of organ (e.g. kidney) is taken from the individuals obtained by mating, the expression level of the polypeptide having aging-suppressing activity or DNA encoding the polypeptide in the organ is determined by Western blotting using an antibody to the polypeptide having aging-suppressing activity or Northern blotting using the DNA encoding the polypeptide as a probe. Then, individuals lacking expression or animals with reduced expression level of the polypeptide are selected.

Specific examples of the homozygous klotho mutant animals include the homozygous klotho mutant mice showing premature aging-like syndrome reported at the 18th Annual Meeting of The Molecular Biology Society of Japan (Nabeshima, et al., 2K-02, 1995). The mice are transgenic mice carrying an introduced foreign gene comprising human elongation factor 1α promoter (EF1 alpha promoter) and Na⁺/H⁺ antiporter ligated to the promoter (Japanese Published Unexamined Patent Application No. 268856/93). By mating of heterozygous individuals obtained by this method, homozygous individuals showing premature aging-like syndrome and heterozygous individuals were obtained. The homozygous individuals are available from Youichi Nabeshima, Tumor Biology, Pathology, Kyoto University Graduate School of Medicine.

Various kinds of heterozygous klotho mutant animals can be obtained by methods similar to the above method, and homozygous klotho mutant animals can be obtained by mating of the heterozygous klotho mutant animals.

In the present invention, symptoms of premature aging-like syndrome of homozygous klotho mutant animals include, for example, in the case of mice, retardation in body weight increase, reduction in life span (less than about 9 weeks), ectopic calcification at arterial media and other tissues, abnormality in osteogenesis such as decrease in bone density, thymic involution, missing of Purkinje cells, infertility by gonadal dysgenesis, skin atrophy accompanied by decrease of subcutaneous fat and hair roots, abnormality in motor functions such as walking, and abnormality in pituitary function, which appear after the weaning period (3 weeks old).

These premature aging-like symptoms can be assessed by measurement of body weight, examination on the acquisition of reproductive ability, measurement of blood sugar level, observation of life span, observation of skin, observation of motor functions such as walking, and the like. The assessment can also be made by measurement of thymus weight, observation of the size of calcified nodules formed on the inner surface of thoracic cavity, and the like. Further, quantitative determination of mRNA for the klotho gene or Klotho protein is also useful for the assessment.

The determination of Klotho protein can be carried out by methods such as immune precipitation, Western blotting and enzyme-linked immunosorbent assay (ELISA) using an anti-Klotho protein antibody, and the determination of mRNA for the klotho gene can be carried out by methods such as Northern blot hybridization and reverse transcription-polymerase chain reaction (RT-PCR). The determination methods and methods for obtaining tissue samples, klotho gene, Klotho protein, probe DNA, primers, antibodies, etc. to be used in the determination are described in WO 98/29544.

The present invention is described in detail below, using for an example the case of mouse.

In the present invention, the premature aging-like syndrome is regarded as being treated or prevented when any one of the following phenomena is caused in homozygous klotho mutant mice.
(1) At the age of 5 weeks, the body weight of homozygous klotho mutant mice reaches more than 60%, preferably more than 70%, more preferably more than 80%, of that of heterozygous klotho mutant mice.
(2) At the age of 10 weeks, the mice show a survival rate of more than 50%, preferably more than 60%, more preferably more than 80%.
(3) By the age of 20 weeks, more than 30%, preferably more than 50%, more preferably more than 80%, of individuals acquire reproductive ability.
(4) The amount of mRNA for klotho gene or Klotho protein expressed in homozygous klotho mutant mice is more than 1/500, preferably more than 1/20, more preferably more than 1/5, of that in wild-type mice of the same age (weeks) fed with a commercially available feed, CE2 (Clea Japan, Inc.).

The therapeutic or preventing agent for diseases caused by a decrease in the expression level of Klotho protein in animals or humans of the present invention comprises ornithine or a salt thereof, and if necessary, may comprise one or more pharmaceutically acceptable carriers, and further, an additional active ingredient for another therapeutic purpose.

The salts of ornithine include acid addition salts, metal salts, ammonium salts, organic amine addition salts and amino acid addition salts.

Examples of the acid addition salts include inorganic acid addition salts such as hydrochloride, sulfate, nitrate and phosphate, and organic acid addition salts such as acetate, maleate, fumarate, citrate, malate, lactate, α-ketoglutarate, gluconate and caprylate. Examples of the metal salts include alkali metal salts such as sodium salt and potassium salt, alkaline earth metal salts such as magnesium salt and calcium salt, aluminum salt and zinc salt. Examples of the ammonium salts are ammonium salt and tetramethylammonium salt. Examples of the organic amine addition salts are salts with morpholine and piperidine. Examples of the amino acid addition salts are salts with glycine, phenylalanine, lysine, aspartic acid and glutamic acid.

The therapeutic or preventing agent for disease caused by a decrease in the expression level of Klotho protein of the present invention is produced according to an arbitrary method well known in the technical field of pharmaceutics by mixing ornithine or a salt thereof with a carrier, as may be required.

It is desirable to administer the preparation by the route that is most effective for the treatment. Suitable administration routes include oral administration and non-oral administration such as intravenous administration, intraperitoneal administration or subcutaneous administration. Preferred is oral administration.

The preparation is administered in the form of tablets, powders, granules, syrup, injection, or the like.

Liquid preparations suitable for oral administration such as syrup can be prepared using carriers such as water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), preservatives (e.g., paraoxybenzoic acid derivatives such as methyl paraoxybenzoate, and sodium benzoate) and flavors (e.g., strawberry flavor and peppermint).

Tablets, powders, granules, etc. suitable for oral administration can be prepared using excipients such as sugars (e.g., lactose, white sugar, glucose, sucrose, mannitol and sorbitol), starch (e.g., potato starch, wheat starch and corn starch), inorganic substances (e.g., calcium carbonate, calcium sulfate, sodium hydrogencarbonate and sodium chloride), fillers such as crystalline cellulose and plant powders (e.g., licorice powder and gentian powder), disintegrators such as starch, agar, gelatin powder, crystalline cellulose, carmellose sodium, carmellose calcium, calcium carbonate, sodium hydrogencarbonate and sodium alginate, lubricants such as magnesium stearate, talc, hydrogenated vegetable oil, macrogol and silicone oil, binders such as polyvinyl alcohol, hydroxypropyl cellulose, methyl cellulose, ethyl cellulose, carmellose, gelatin and starch paste, surfactants such as fatty acid esters, plasticizers such as glycerin, and the like.

Preparations suitable for non-oral administration such as injection preferably comprise a sterilized aqueous preparation containing an active compound which is isotonic to the recipient's blood. In the case of an injection, for example, a solution for injection is prepared using a carrier comprising a salt solution, a glucose solution, or a mixture of salt water and a glucose solution.

The above-described antiseptics, preservatives, surfactants, etc. can also be employed in non-oral preparations.

When the therapeutic or preventing agent for diseases caused by a decrease in the expression of Klotho protein of the present invention is administered to a human, the dose and administration schedule will vary depending on the administration route, the age and body weight of a patient, and the symptom and degree of the disease to be treated, without specific restriction. Usually, in the case of oral administration, the agent is administered in a dose of 0.01 mg to 50 g, preferably 0.05 mg to 10 g in terms of ornithine or a salt thereof per adult once to several times per day. In the case of non-oral administration such as intravenous administration, the agent is administered in a dose of 0.001 mg to 50 g, preferably 0.01 mg to 10 g in terms of ornithine or a salt thereof per adult once to several times per day.

When the therapeutic or preventing agent for diseases caused by a decrease in the expression level of Klotho protein of the present invention is administered to an animal, the dose and administration schedule will vary depending on the age and kind of the animal and the symptom and degree of the disease, without specific restriction. In the case of oral administration, the agent is administered in a dose of 0.1 µg to 10 g, preferably 1 µg to 1 g per kg of body weight in terms of ornithine or a salt thereof once to several times per day. In the case of non-oral administration such as intravenous administration, the agent is administered in a dose of 0.01 µg to 10 g, preferably 1 µg to 1 g per kg of body weight in terms of ornithine or a salt thereof once to several times per day. However, the dose and administration schedule may vary depending upon various conditions as given above.

The therapeutic or preventing agent for diseases caused by a decrease in the expression level of Klotho protein of the present invention is especially preferred for use in homozygous klotho mutant animals. By administering the agent to homozygous klotho mutant animals, their diseases caused by a decrease in the expression level of Klotho protein can be treated or prevented.

The feed or food and drink for the treatment or prevention of diseases caused by a decrease in the expression level of Klotho protein in animals or humans which comprises ornithine or a salt thereof as an active ingredient includes feeds or foods and drinks originally containing ornithine or a salt thereof, as well as those produced by adding ornithine or a salt thereof to feeds or foods and drinks which do not contain ornithine or a salt thereof in an ordinary process for the production of feeds or foods and drinks.

The feed or food and drink obtainable by adding ornithine or a salt thereof may be processed by molding and granulating methods. The molding and granulating methods include granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method, and extrusion methods using an extruding granulator and an extruder.

The content of ornithine or a salt thereof in the feed or food and drink is not specifically limited, so far as the feed or food and drink can exert the therapeutic or preventing action on diseases caused by a decrease in the expression level of Klotho protein in animals or humans. The content of ornithine or a salt thereof in the feed or food and drink is preferably 0.001 to 100 wt%, more preferably 0.01 to 100 wt%, particularly preferably 0.1 to 100 wt%.

Examples of the foods and drinks of the present invention are juice, soft drinks, soup, tea, dairy products (e.g., lactic acid bacteria beverages, fermented milk, ice cream, butter, cheese, yogurt, processed milk and skim milk), meat products (e.g., ham, sausage and hamburger), fish paste products, egg products (e.g., baked or steamed foods made of beaten eggs), confectionery (e.g., cookies, jelly, snacks and chewing gum), bread, noodles, pickles, smoked fish and meat, dried fish, preserved foods boiled down with soy and seasonings which comprise ornithine or a salt thereof.

The food and drink may be in any of the forms such as a powder food, a sheet-shaped food, a bottled food, a canned food, a retort pouched food, a capsule food, a tablet food, a liquid food and a liquid nutrient food.

The food and drink of the present invention is used as a health food and drink or a functional food and drink for the treatment or prevention level of diseases caused by a decrease in the expression of Klotho protein.

There is no specific restriction as to the intake of the food and drink for the treatment or prevention of diseases caused by a decrease in the expression level of Klotho protein in humans which comprises ornithine or a salt thereof as an active ingredient of the present invention. It is generally suitable to take ornithine or a salt thereof in an amount of 0.01 mg to 50 g, preferably 0.05 mg to 10 g per adult per day for the period of one day to one year, preferably 2 weeks to 3 months. This intake is merely a typical example and can be appropriately adjusted according to the degree of the recipient's condition, age, body weight, etc.

The feed of the present invention may be any feed comprising ornithine or a salt thereof as an active ingredient, for example, a feed obtained by adding ornithine or a salt thereof to an ordinary feed or feed materials.

The feed includes a feed for pets such as dogs, cats and mice, a feed for livestock such as cows and pigs, a feed for poultry such as hens and turkeys, and a feed for cultivated fish such as sea breams and young yellowtails.

A specific example of the feed to which ornithine or a salt thereof is to be added is a feed designed to contain 10 to 70 wt% protein, 0 to 50 wt% fat and oil, 5 to 80 wt% carbohydrate, 0.1 to 5 wt% vitamin mixture, 0.5 to 15 wt% mineral mixture, and 0 to 20 wt% fiber.

Examples of the proteins include vegetable proteins derived from beans such as soybean, cereal grains such as corn, and potatoes such as white potato, and animal proteins such as albumin and globulin derived from poultry egg, casein and whey derived from livestock milk, collagen derived from poultry and livestock meat, protein derived from poultry and livestock meat, fish and shell, and globulin and albumin derived from poultry and livestock blood.

Examples of the fats and oils include vegetable oils derived from corn, soybean, safflower, canola, rapeseed, olive, sesame, sunflower, cottonseed, peanut, coconut, cacao, rice, camellia, cuphea, palm, etc., and animal fats and oils derived from meat and aquatic products (e.g., lard, tallow, chicken oil, mutton oil, fish oil and whale oil) and milk (e.g., butter).

Examples of the carbohydrates include starch derived from potatoes such as white potato and sweet potato, starch derived from cereal grains such as corn, rice and wheat, starch hydrolyzate, disaccharides such as maltose, sucrose and lactose, and monosaccharides such as fructose, glucose, galactose, arabinose, mannose and xylose.

Examples of the fibers include glucan, xylan, mannan, galactan and their derivatives included in cellulose and hemicellulose, seaweed-derived dietary fibers such as alginic acid, carrageenan and furcellaran, gum such as polyuronide, hardly digestible components of dextrin, galacturonan, lignin, chitin, chitosan, psyllium, and their hydrolyzates.

Examples of the minerals include compounds containing calcium, phosphorus, magnesium, potassium, sodium, manganese, iron, copper, zinc, cobalt, iodine, selenium, chromium, bromine, fluorine, molybdenum, vanadium, nickel and lithium.

Examples of the vitamins include vitamin A, vitamin D₃ (cholecalciferol), vitamin E, vitamin B₁, vitamin B₂, vitamin B₆, vitamin B₁₂, nicotinic acid, pantothenic acid, folic acid, biotin and vitamin K₃.

The feed of the present invention preferably contains a substance inhibiting or promoting the gastrointestinal absorption or renal resorption of phosphorus, and a steroid.

The feed materials include cereal grains, bran, vegetable oil cakes, animal-derived feed materials, other feed materials and purified products.

Examples of the cereal grains are milo, wheat, barley, oats, rye, brown rice, buckwheat, foxtail millet, broomcorn millet, Japanese millet, corn and soybean.

Examples of the bran are rice bran, defatted rice bran, wheat bran, wheat middlings, wheat germ, screenings, pellets, corn bran and corn germ.

Examples of the vegetable oil cakes are soybean cake, soybean flour, linseed cake, cottonseed cake, peanut cake, safflower cake, coconut cake, palm cake, sesame cake, sunflower cake, rapeseed cake, kapok cake and mustard seed cake.

Examples of the animal-derived feed materials are fish meal (e.g., white meal, imported meal, whole meal and brown meal), fish soluble, meat meal, meat and bone meal, blood powder, degradated hair, bone meal, by-products of processing of livestock products, feather meal, silkworm pupa, skim milk, casein and dry whey.

Examples of other feed materials are stalks and leaves of plants (e.g., alfalfa, hay cube, alfalfa leaf meal and powder of false acacia), by-products of industrial processing of corn (e.g., corn gluten, meal, corn gluten feed and corn steep liquor), processed starch products (e.g., starch), sugar, fermentation industrial products (e.g., yeast, beer cake, malt root, alcohol cake and soy sauce cake), agricultural by-products (e.g., citrus fruit cake, tofu cake, coffee cake and cocoa cake) and others (e.g., cassava, broad bean, guar meal, seaweeds, krill, spirulina, chlorella and minerals).

Examples of the purified products are proteins (e.g., casein and albumin), amino acids, carbohydrates (e.g., starch, cellulose, sucrose and glucose), minerals and vitamins.

There is no specific restriction as to the intake of the feed for the treatment or prevention of diseases caused by a decrease in the expression level of Klotho protein which comprises ornithine or a salt thereof as an active ingredient of the present invention. It is generally suitable to allow an animal to take ornithine or a salt thereof in an amount of 0.1 mg to 50 g, preferably 0.5 mg to 10 g per kg of body weight per day for the period of one day to one year, preferably 2 weeks to 3 months. This intake is merely a typical example and can be appropriately adjusted according to the kind, age, body weight, etc. of the animal.

The feed or food and drink of the present invention preferably comprises at least one compound selected from the group consisting of compounds containing a divalent cationic metal and compounds containing phosphorus.

Preferred divalent cationic metals include calcium, magnesium and zinc, among which magnesium and zinc are more preferred and zinc is particularly preferred.

The compounds containing such metals include organic metal salts and inorganic metal salts. Examples of the organic metal salts include metal salts of organic acids (e.g., orotic acid) such as zinc orotate, and examples of the inorganic metal salts include halogenated metal salts such as magnesium chloride and zinc chloride.

The compounds containing phosphorus include inorganic phosphates and phosphoric esters. Examples of the inorganic phosphates are phosphate (PO₄³⁻), hydrogenphosphate (HPO₄²⁻), dihydrogenphosphate (H₂PO₄⁻), diphosphate (P₂O₇⁴⁻), dihydrogendiphosphate (H₂O₇P₂²⁻), triphosphate (P₃O₁₀⁵⁻), tetraphosphate (P₄O₁₃⁶⁻), metaphosphate (PO₃⁻), phosphite (HPO₃²⁻), hypophosphate (P₂O₆⁴⁻), hypophosphite (PH₂O₂⁻) and hydroxyapatite. Examples of the phosphoric esters are adenosine triphosphate releasing inorganic phosphoric acid when hydrolyzed, and phytic acid.

The concentration of the compound containing a divalent cationic metal in the feed or food and drink may vary according to the concentrations of other components (e.g., phosphorus), but is preferably 0.001 to 5 wt%, more preferably 0.01 to 2 wt%, particularly preferably 0.1 to 1 wt%, in terms of metal weight per unit dry weight of feed or food and drink. For the purpose of treatment or prevention of diseases caused by a decrease in the expression level of Klotho protein, the concentration is preferably 0.1 to 5 wt%, more preferably 0.2 to 2 wt%.

The concentration of the compound containing phosphorus in the feed or food and drink may vary according to the concentrations of other components (e.g., a divalent cationic metal), but is preferably 0.001 to 5 wt%, more preferably 0.01 to 2 wt%, particularly preferably 0.1 to 1 wt%, in terms of phosphorus weight per unit dry weight of feed or food and drink. For the purpose of treatment or prevention of diseases caused by a decrease in the expression level of Klotho protein, the concentration is preferably 0.001 to 1.0 wt%, more preferably 0.01 to 0.6 wt%.

Especially, for the purpose of treatment or prevention of diseases caused by a decrease in the expression level of Klotho protein, the concentration of phosphorus in the form of inorganic phosphate (PO₄³⁻) is preferably 0.001 to 0.8 wt%, more preferably 0.01 to 0.7 wt%, particularly preferably 0.1 to 0.65 wt%, based on dry weight of feed or food and drink.

The concentrations of divalent cationic metal and phosphorus in the feed or food and drink are expressed in weight *per* cent based on dry weight of feed or food and drink. The concentrations of metal and phosphorus in the feed or food and drink can be measured according to known methods.

The feed for the treatment or prevention of diseases caused by a decrease in the expression level of Klotho protein in animals which comprises ornithine or a salt thereof as an active ingredient of the present invention is especially preferred as a feed for homozygous klotho mutant animals. By feeding homozygous klotho mutant animals with the feed, their premature aging-like syndrome can be treated or prevented.

The therapeutic or preventing agent for premature aging-like syndrome of homozygous klotho mutant animals and the feed for the treatment or prevention of premature aging-like syndrome of homozygous klotho mutant animals can be used for the purpose of treatment or prevention of premature aging-like syndrome of homozygous klotho mutant animals, for example, for prolonging the life span of homozygous klotho mutant animals, for inhibiting or alleviating aging-like syndrome characteristic of homozygous klotho mutant animals, for conferring reproductive ability on homozygous klotho mutant animals, and for regulating the expression level of Klotho protein in homozygous klotho mutant animals.

The present invention provides homozygous klotho mutant animals with prolonged life span, homozygous klotho mutant animals whose premature aging-like syndrome has been inhibited or alleviated, homozygous klotho mutant animals which acquired reproductive ability, and homozygous klotho mutant animals in which expression level of Klotho protein is regulated. The present invention also provides aged (mature) homozygous klotho mutant animals manifesting premature aging-like syndrome after the syndrome has been once inhibited or alleviated by the above method. These animals are all useful, as well as homozygous klotho mutant animals showing premature aging-like syndrome, in experiments for studies on the methods for treating or preventing aging syndrome, the analysis of aging mechanism, the analysis of mechanism of klotho gene or Klotho protein, and the like.

The method for treating or preventing premature aging-like syndrome of homozygous klotho mutant animals according to the present invention can be carried out by feeding the animals with the therapeutic or preventing agent or feed of the present invention and if necessary, another feed and water in the methods for raising or breeding normal animals of the same kind as the subject animals. It is preferable to feed animals with the feed of the present invention continuously from the weaning period. Homozygous individuals of klotho mutant animals raised on the feed of the present invention to reach maturity acquire reproductive ability, and therefore, breeding of homozygous individuals can be effected by mating of pairs of such homozygous individuals.

By feeding animals with the therapeutic or preventing agent or feed of the present invention from the weaning period (age of 3 weeks) for one week, the growth of animals thereafter can be fully expected. Further, once homozygous klotho mutant animals have acquired reproductive ability, they need not always be fed with the therapeutic or preventing agent or feed of the present invention and may be fed with an ordinary feed.

The therapeutic or preventing agent or feed of the present invention is fed to subject animals preferably for the period of 2 days after weaning, more preferably for one week after weaning, particularly preferably until the period at which normal animals of the same kind as the subject homozygous klotho mutant animals become capable of reproduction after weaning. For example, mice usually become capable of reproduction at the age of about 8 weeks, and it is further preferred to feed mice with the feed of the present invention until the age of 15 weeks after weaning. Homozygous klotho mutant animals which have acquired reproductive ability are genetically homozygous and their offspring are all homozygous klotho mutant animals. Accordingly, they can be preferably used as pairs for breeding. Further, by feeding of a feed capable of inducing or promoting aging-like syndrome of homozygous klotho mutant animals, aged (mature) homozygous klotho mutant animal models of aging-like syndrome are provided.

The "feed capable of inducing or promoting aging-like syndrome of homozygous Klotho mutant animals" may be any feed which is capable of inducing or promoting aging-like syndrome of homozygous Klotho mutant such as CE2, OA2(CLEA Japan, Inc.) and a powder feed obtainable by uniformly mixing with 20 parts by weight of casein, 5 parts by weight of lard, 1 part by weight of corn-oil, 20 parts by weight of sucrose, 5 parts by weight of cellulose, 0.2 part by weight of choline chloride, 1 part by weight of vitamine mixture which consists of the same composition as AIN-76, 4.85 parts by weight of potassium dihydrogenphosphate, 3.5 parts by weight of mineral mixture which consists of the same composition as AIN-76 and 39.45 parts by weight of corn starch.

The "animals in which expression of Klotho protein is regulated or development of aging-like syndrome is controlled" according to the present invention refers to the following: aged (mature) animals showing a decrease in Klotho protein or developing klotho mutant symptoms, which are obtained by raising klotho mutant animals usually incapable of growing to maturity on the feed capable of inhibiting or alleviating aging-like syndrome of homozygous klotho mutant animals for a certain period of time and then feeding the animals with a feed capable of inducing or promoting aging-like syndrome of homozygous klotho mutant animals; animals which have recovered from klotho mutant symptoms by feeding of the feed capable of inhibiting or alleviating aging-like syndrome of homozygous klotho mutant animals after the symptoms once appeared; or wild-type or heterozygous klotho mutant animals showing a decreased or increased expression level of klotho gene, which are obtained by feeding of various combinations of feeds capable of inhibiting or alleviating aging-like syndrome of homozygous klotho mutant animals and those capable of inducing or promoting the syndrome for various periods of time.

By changing the feed components and varying the timing of the change, the expression level of Klotho protein, the degree of the syndrome, the life span, etc. of animals can be variously adjusted.

Certain embodiments of the present invention are illustrated in the following examples. In the examples, products of the following manufacturers were used:
lard (Kishida Chemical Co., Ltd.), corn oil (Nacalai Tesgue, Inc.), sucrose (Kishida Chemical Co., Ltd.), cellulose (Oriental Yeast Co., Ltd.), casein (Oriental Yeast Co., Ltd.), choline chloride (Kishida Chemical Co., Ltd.), vitamin mixture (AIN-76, Oriental Yeast Co., Ltd.), mineral mixture (AIH-76, Oriental Yeast Co., Ltd.), corn starch (Kishida Chemical Co., Ltd.), calcium dihydrogenphosphate (Kishida Chemical Co., Ltd.).

### Example 1

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride, 1 part by weight of vitamin mixture, 2 parts by weight of ornithine hydrochloride and 0.9 part by weight of potassium dihydrogenphosphate in a mortar. Then, 3.5 parts by weight of mineral mixture, the remainder of 20 parts by weight of casein and 41.4 parts by weight of corn starch were uniformly mixed together with the dilution obtained above and the mixture obtained above using an automatic mixer for about 10 minutes to prepare a powdery feed.

The obtained feed contains 0.77 wt% phosphorus.

### Comparative Example 1

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride, 1 part by weight of vitamin mixture and 0.9 part by weight of potassium dihydrogenphosphate in a mortar. Then, 3.5 parts by weight of mineral mixture, the remainder of 20 parts by weight of casein and 43.4 parts by weight of corn starch were uniformly mixed together with the dilution obtained above and the mixture obtained above using an automatic mixer for about 10 minutes to prepare a powdery feed.

The obtained feed contains 0.6 wt% phosphorus, most of which is derived from calcium hydrogenphosphate (CaHPO₄) in the mineral mixture and potassium dihydrogenphosphate.

### Example 2

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride, 1 part by weight of vitamin mixture and 2 parts by weight of ornithine hydrochloride in a mortar. Then, 3.5 parts by weight of mineral mixture, the remainder of 20 parts by weight of casein and 41.4 parts by weight of corn starch were uniformly mixed together with the dilution obtained above and the mixture obtained above using an automatic mixer for about 10 minutes to prepare a powdery feed.

The obtained feed contains 0.57 wt% phosphorus, most of which is derived from calcium hydrogenphosphate (CaHPO₄) in the mineral mixture.

### Comparative Example 2

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride and 1 part by weight of vitamin mixture in a mortar. Then, 3.5 parts by weight of mineral mixture, the remainder of 20 parts by weight of casein and 44.3 parts by weight of corn starch were uniformly mixed together with the dilution obtained above and the mixture obtained above using an automatic mixer for about 10 minutes to prepare a powdery feed.

The obtained feed contains 0.57 wt% phosphorus.

### Example 3

Mice carrying a klotho mutation were bred by mating a pair of heterozygous klotho mutant mice provided by Tumor Biology, Pathology, Kyoto University Graduate School of Medicine. Klotho mutant homozygotes, klotho mutant heterozygotes and wild-type mice were confirmed by PCR. Male klotho mutant homozygotes were raised on the feed prepared in Example 1 or the feed prepared in Comparative Example 1 and were intermittently weighed (number of animals: 4/group). The mice were given the powdery feed through a commercially available powder feeder (KN-675 No. 4, Natsume Seisakusho) and filtered running water, both ad libitum. The mice were raised in an SPF (specific pathogen-free) room kept at a temperature of 24 ± 1°C and a humidity of 55 ± 10%. The results are shown in Table 1. The expression level of Klotho protein was expressed as relative values based on the average value of the group raised on the feed of Comparative Example 1 up to the age of 6 weeks as 100.

**Table 1**

| Feed | Age (weeks) | Number of animals | Index of syndrome | Measurement |
|---|---|---|---|---|
| Ex. 1 | 11 | 4 | Blood sugar (mg/dl) | 178 ± 14 |
| | | | Serum inorganic phosphorus (mg/dl) | 10.5 ± 0.5 |
| | | | Final body weight (g) | 21.6 ± 1.7 |
| | | | Relative expression level of Klotho protein | 231 ± 55 |
| Comp. Ex. 1 | 11 | 4 | Blood sugar (mg/dl) | 156 ± 31 |
| | | | Serum inorganic phosphorus (mg/dl) | 13.0 ± 1.7 |
| | | | Final body weight (g) | 17.7 ± 1.9 |
| | | | Relative expression level of Klotho protein | 189 ± 119 |
| Ex. 1 | 6 | 4 | Blood sugar (mg/dl) | 200 ± 31 |
| | | | Serum inorganic phosphorus (mg/dl) | 14.3 ± 1.6 |
| | | | Final body weight (g) | 18.7 ± 0.5 |
| | | | Relative expression level of Klotho protein | 188 ± 38 |
| Comp. Ex. 1 | 6 | 4 | Blood sugar (mg/dl) | 164 ± 14 |
| | | | Serum inorganic phosphorus (mg/dl) | 17.1 ± 2.6 |
| | | | Final body weight (g) | 15.0 ± 3.2 |
| | | | Relative expression level of Klotho protein | 100 ± 75 |

At the age of 6 weeks, the group of mice raised on the feed of Comparative Example 1 containing no ornithine showed the following values: blood sugar, 164 ± 14 mg/dl; serum inorganic phosphorus, 17.1 ± 2.6 mg/dl; and body weight, 15.0 ± 3.2 g. On the other hand, those values of the group of mice raised on the feed of Example 1 containing ornithine were 200 ± 31 mg/dl, 14.3 ± 1.6 mg/dl, and 18.7 ± 0.5 g, respectively. That is, the body weight and the blood sugar level were higher and the serum inorganic phosphorus level was lower in the latter group, suggesting that the development of premature aging-like syndrome was inhibited. The expression level of Klotho protein in the kidneys was 100 ± 75 in the group fed on the feed of Comparative Example 1 containing no ornithine and was increased to 188 ± 38 in the group fed on the feed of Example 1 containing ornithine.

A similar tendency was observed at the age of 11 weeks. That is, the group of mice raised on the feed of Comparative Example 1 containing no ornithine showed the following values: blood sugar, 156 ± 31 mg/dl; serum inorganic phosphorus, 13.0 ± 1.7 mg/dl; and body weight, 17.7 ± 1.9 g. On the other hand, those values of the group of mice raised on the feed of Example 1 containing ornithine were 178 ± 14 mg/dl, 10.5 ± 0.5 mg/dl, and 21.6 ± 1.7 g, respectively, suggesting that the development of premature aging-like syndrome was inhibited. The expression level of Klotho protein in the kidneys was 189 ± 119 in the group fed on the feed of Comparative Example 1 containing no ornithine and was increased to 231 ± 55 in the group fed on the feed of Example 1 containing ornithine.

The above results indicated that ornithine was effective in raising the expression level of kidney Klotho protein, raising the blood sugar level, lowering the serum inorganic phosphorus level and increasing the body weight of male homozygous klotho mutant mice, i.e., ornithine had inhibitory effects on premature aging-like syndrome of the mice.

### Example 4

Female klotho mutant homozygotes obtained by the same method as in Example 3 were raised on the feed of Example 1 or the feed of Comparative Example 1 in the same manner as in Example 3 up to the age of 8 weeks, and their body weight was measured. The body weight of the group fed on the feed of Comparative Example 1 was 10.9 ± 0.7 g, whereas that of the group fed on the feed of Example 1 was 14.1 ± 1.7 g. That is, the mice of the group fed on the feed containing ornithine had a significantly higher body weight (t test, P<0.05). The weight-increasing effect of ornithine was thus observed also on female individuals.

### Example 5

Female klotho mutant homozygotes obtained by the same method as in Example 3 were raised on the feed of Example 2 or the feed of Comparative Example 2 in the same manner as in Example 3 up to the age of 6 weeks, and their body weight was measured. The body weight of the group fed on the feed of Example 2 was 16.5 ± 0.7 g, whereas that of the group fed on the feed of Comparative Example 2 was 13.6 ± 1.0 g. That is, the mice of the group fed on the feed containing ornithine had a significantly higher body weight (t test, P<0.01). Similarly to Example 4, the weight-increasing effect of ornithine was observed on female homozygous individuals.

### Example 6

Female klotho mutant homozygotes obtained by the same method as in Example 3 were raised on the feed of Example 1, the feed of Comparative Example 1, the feed of Example 2 or the feed of Comparative Example 2 (number of animals: 4/group) to observe their life span. The number of mice that died before reaching the age of 11 weeks was 0 and 2 in the groups fed on the feed of Example 1 and the feed of Comparative Example 1, respectively, and was 1 and 2 in the groups fed on the feed of Example 2 and the feed of Comparative Example 2, respectively.

The number of deaths was decreased in the groups fed on the feeds containing ornithine as shown above, indicating that ornithine was effective in prolonging life span.

### Example 7

A vitamin mixture of special composition based on AIN-76 was prepared by gradually diluting 13.76 parts by weight of vitamin A acetate, 500 parts by weight of vitamin E acetate, 0.5 part by weight of vitamin K₃, 60 parts by weight of vitamin B₁ hydrochloride, 60 parts by weight of vitamin B₂, 70 parts by weight of vitamin B₆ hydrochloride, 0.1 part by weight of vitamin B₁₂, 2 parts by weight of D-biotin, 20 parts by weight of folic acid, 160 parts by weight of calcium pantothenate and 300 parts by weight of nicotinic acid with 98814 parts by weight of sucrose in a mortar and uniformly mixing all components.

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride, 1 part by weight of the above vitamin mixture of special composition and 2 parts by weight of ornithine hydrochloride in a mortar. Then, 3.5 parts by weight of mineral mixture, the remainder of 20 parts by weight of casein and 42.3 parts by weight of corn starch were uniformly mixed together with the dilution obtained above and the mixture obtained above using an automatic mixer for about 10 minutes to prepare a powdery feed.

### Comparative Example 3

Five parts by weight of lard melted in a hot water bath at 60°C, 1 part by weight of corn oil, 20 parts by weight of sucrose and 5 parts by weight of cellulose were uniformly mixed in a bowl using a rubber spatula. About a half of 20 parts by weight of casein was used to dilute 0.2 part by weight of choline chloride, 0.25 part by weight of zinc orotate and 1 part by weight of vitamin mixture of AIN-76 composition in a mortar. Then, 3.5 parts by weight of mineral mixture, the remainder of 20 parts by weight of casein and 44.05 parts by weight of corn starch were uniformly mixed together with the dilution obtained above and the mixture obtained above using an automatic mixer for about 10 minutes to prepare a powdery feed.

### Example 8

Male homozygous klotho mutant mice were raised on the feed of Comparative Example 3 and female homozygous klotho mutant mice were raised on the feed of Example 7 or the feed of Comparative Example 2, both to the age of 8 weeks. These male and female mice were mated and their breeding results were compared between the female group fed on the feed of Example 7 and that fed on the feed of Comparative Example 2. During the mating period, the males were transferred into the cages of females and so fed on the same feeds as the respective females.

Four females fed on the feed of Comparative Example 2 became pregnant and bred 16 mice in total. However, all of the newborn mice died within 2 days after birth. That is, the weaning rate based on the total number of newborns was 0% in the group fed on the feed of Comparative Example 2. On the other hand, 6 females fed on the feed of Example 7 became pregnant and bred 12 mice in total. Of these 12 newborns, 8 grew to the age of 3 weeks and reached the weaning period, giving the weaning rate of 67%. Of the 6 mother mice in this group 3 succeeded in growing all of the litter to reach the weaning period.

The above results demonstrated that the feeding of ornithine improved the breeding result of female mice.

## Claims

1. A therapeutic or preventing agent for a disease caused by a decrease in the expression level of Klotho protein in an animal or a human which comprises ornithine or a salt thereof as an active ingredient.

2. The therapeutic or preventing agent according to Claim 1, wherein the disease is an aging-associated disease.

3. A feed or food or drink for the treatment or prevention of a disease caused by a decrease in the expression level of Klotho protein in an animal or a human which comprises ornithine or a salt thereof as an active ingredient.

4. The feed or food or drink according to Claim 3, further comprising at least one compound selected from the group consisting of compounds containing a divalent cationic metal and compounds containing phosphorus.

5. The feed or food or drink according to Claim 3 or 4, wherein the disease is an aging-associated disease.

6. A therapeutic or preventing agent for premature aging-like syndrome of a homozygous klotho mutant animal which comprises ornithine or a salt thereof as an active ingredient.

7. A feed for the treatment or prevention of premature aging-like syndrome of a homozygous klotho mutant animal which comprises ornithine or a salt thereof as an active ingredient.

8. The feed according to Claim 7, further comprising at least one compound selected from the group consisting of compounds containing a divalent cationic metal and compounds containing phosphorus.

9. A method for treating or preventing premature aging-like syndrome of a homozygous klotho mutant animal which comprises feeding the animal with the feed according to Claim 7 or 8.

10. The method according to Claim 9, wherein the animal is a homozygous klotho mutant mouse.

11. Use of ornithine or a salt thereof for the preparation of a therapeutic or preventing agent for a disease caused by a decrease in the expression level of Klotho protein in an animal or a human.

12. Use of ornithine or a salt thereof for the preparation of a feed or food or drink for a disease caused by a decrease in the expression level of Klotho protein in an animal or a human.
